(19) Europäisches Patentamt / European Patent Office / Office européen des brevets

(11) **EP 3 375 456 B1**

(12) **EUROPEAN PATENT SPECIFICATION**

(45) Date of publication and mention
of the grant of the patent:
**03.05.2023 Bulletin 2023/18**

(21) Application number: **16864084.5**

(22) Date of filing: **01.11.2016**

(51) International Patent Classification (IPC):
*A61K 9/00* (2006.01)    *A61K 9/06* (2006.01)
*A61K 47/02* (2006.01)    *A61K 47/36* (2006.01)
*A61P 5/00* (2006.01)     *A61P 9/00* (2006.01)
*A61P 29/00* (2006.01)    *A61P 25/02* (2006.01)
*A61P 31/00* (2006.01)    *A61P 31/04* (2006.01)
*A61P 31/10* (2006.01)    *A61P 31/12* (2006.01)
*A61P 35/00* (2006.01)    *A61P 43/00* (2006.01)
*A61K 31/445* (2006.01)   *A61P 23/02* (2006.01)

(52) Cooperative Patent Classification (CPC):
**A61K 31/445; A61K 9/0014; A61K 9/06;**
**A61K 47/36; A61P 5/00; A61P 9/00; A61P 23/02;**
**A61P 25/02; A61P 29/00; A61P 31/00;**
**A61P 31/04; A61P 31/10; A61P 31/12;**
**A61P 35/00; A61P 43/00;**                    (Cont.)

(86) International application number:
**PCT/JP2016/082456**

(87) International publication number:
**WO 2017/082121 (18.05.2017 Gazette 2017/20)**

(54) **SUSTAINED-RELEASE TOPICALLY ADMINISTERED AGENT**

TOPISCH VERABREICHTES MITTEL MIT VERZÖGERTER FREISETZUNG

AGENT ADMINISTRÉ PAR VOIE TOPIQUE À LIBÉRATION PROLONGÉE

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **12.11.2015 JP 2015222512**

(43) Date of publication of application:
**19.09.2018 Bulletin 2018/38**

(73) Proprietor: **Terumo Kabushiki Kaisha**
**Tokyo 151-0072 (JP)**

(72) Inventors:
• **KIKUCHI, Hideka**
  **Ashigarakami-gun**
  **Kanagawa 259-0151 (JP)**
• **IWAKIRI, Chisato**
  **Ashigarakami-gun**
  **Kanagawa 259-0151 (JP)**

(74) Representative: **Casalonga**
**Casalonga & Partners**
**Bayerstraße 71/73**
**80335 München (DE)**

(56) References cited:
EP-A1- 1 595 892         WO-A1-2004/050712
WO-A1-2009/133763        WO-A2-2010/043979
JP-A- H06 508 169        JP-A- 2008 169 313
JP-A- 2008 542 295       US-A1- 2008 058 469

EP 3 375 456 B1

(52) Cooperative Patent Classification (CPC): (Cont.)
A61K 47/02

## Description

TECHNICAL FIELD

The invention relates to a sustained-release topically administered agent. In other words, the invention relates to a sustained-release medical agent for local administration.

BACKGROUND ART

There is a need for a sustained-release topically administered agent which can be used for administration to a part of a body and has a property of releasing a compound (medication) having a pharmacological action in a certain period of time and a property that an entire administered agent including a dosage form disappears without putting time after the medication release.

As such a sustained-release topically administered agent, a local anesthetic agent containing a local anesthetic medication is conceivable. The local anesthetic medication is generally used to restrain nociceptive pain. Normally, the local anesthetic medication is administered by a local injection. Normally, a pharmaceutical composition for use in the local injection contains the local anesthetic medication having a concentration of 0.2 to 2%.

In recent years, it is known that owing to a change of the balance between risk and benefit caused by a growing trend of the standardization of a postoperative anticoagulant therapy and a growing trend of allowing a patient to have minimal invasion in a surgical operation, it is difficult to use epidural anesthesia which has been regarded as a gold standard as a first selection of postoperative pain relief.

As an alternate method of the epidural anesthesia, currently opioid IV-PCA and peripheral nerve block and the combination thereof are mainly used. Although postoperative analgesic effect is demanded to continue for one to three days, the duration of effect of the peripheral nerve block caused by the injection of the local anesthetic medication is as short as half a day at the longest.

Recently gelling local anesthetic agents containing the local anesthetic medication are proposed. The gelling local anesthetic agent has the property of gradually releasing the medication and of disappearing the gel over time to some extent. As the gelling local anesthetic agent, there is disclosed in a patent document 1 (Japanese translation of PCT International Application Publication No. 2011-508788, WO2009-129149) a plurality of the implantable drug depot preparations useful for decreasing, preventing or treating pain and/or inflammation of a patient required to be treated. The implantable drug depot preparations include not less than one depot preparation of the first set capable of releasing the analgesic agent and/or the anti-inflammatory agent having the therapeutically effective bolus amount or the salts thereof pharmaceutically acceptable at a subcutaneous part. The implantable drug depot preparations further include not less than one depot preparation of the second set capable of releasing the analgesic agent and/or the anti-inflammatory agent having the therapeutically effective amount or salts thereof pharmaceutically acceptable for at least three days.

There is disclosed in a patent document 2 (Japanese translation of PCT International Application Publication No. 2013-523693, WO2011-121074) the thermal gelation stabilizing pharmaceutical composition, containing at least one kind of the local anesthetic medication, which has the pH close to the pKa of the local anesthetic medication. The thermal gelation stabilizing pharmaceutical composition includes (a) the salt form of at least one kind of the amide-type local anesthetic medication (ATC code: N01BB), (b) 10 to 30 % by weight of the polyoxyethylene castor oil, and (c) 15% by weight of at least one kind of the surface-active agent for imparting the thermal gelation property to the thermal gelation stabilizing pharmaceutical composition.

There is disclosed in a patent document 3 (Japanese translation of PCT International Application Publication No. 2013-523694, WO2011-121082, US Patent Application Publication No. 2013-079371) the stabilized bioadhesive gelling aqueous pharmaceutical composition, having the anisotropic organic phase behavior enabling an administered part containing excessive moisture to swell, which includes (a) not less than one kind of the local anesthetic medication having the anesthesia effective amount, (b) 15 to 70 % by weight of monoglyceride or diglyceride or the mixture of long-chain fatty acid thereof, and (c) 5 to 60 % by weight of the free long-chain saturated fatty acid or the unsaturated fatty acid.

There is disclosed in a patent document 4 (Japanese translation of PCT International Application Publication No. 2006-523731, WO2004-92223) the crosslinked polysaccharide gel (epoxy crosslinked saccharide) improved in its decomposition property (improved in resistance to decomposition of gel) and being useful for pharmaceutical application. It is disclosed in the paragraph number 0043 of the patent document 4 that the crosslinked polysaccharide gel can be combined with a local anesthetic medication.

There is proposed and disclosed in a patent document 5 (Japanese translation of PCT International Application Publication No. 2007-521225, WO2005-009408) the sustained release formulation which releases anesthetic agents such as bupivacaine for a short period of time and is thus used for pain relief of post-operative wound. There is also disclosed therein the formulation dissolved or dispersed in the gel excipient and containing the anesthetic agent. As the gel excipient, the polysaccharide containing an ester end group is exemplified. As one of the anesthetic agents, ropivacaine is proposed. The anesthetic agents of the patent document 5 consist of the viscous gel formed of the polymer and the solvent.

The present applicant proposed the adhesion prevention agent in a patent document 6 (International Publication WO2005-087289, USP 7737214). The adhesion prevention agent consists of the crosslinking polysaccharide derivative having at least one active ester group, introduced into a side chain of a polysaccharide, which is capable of reacting with an active hydrogen-containing group, and being capable of forming the crosslinked substance by the covalent bond between the active ester group and the active hydrogen-containing group owing to the contact between the crosslinking polysaccharide derivative and water under an alkaline condition.

There is disclosed in patent document 8 (WO 2010/043979 A2) an implant comprising polysaccharide hydrogel precursors comprising an active agent for being released.

PRIOR ART DOCUMENT

PATENT DOCUMENT

Patent document 1
Japanese translation of PCT International Application Publication No. 2011-508788 (WO2009-129149)
Patent document 2
Japanese translation of PCT International Application Publication No. 2013-523693 (WO2011-121074)
Patent document 3
Japanese translation of PCT International Application Publication No. 2013-523694 (WO2011-121082, US Patent Application Publication No. 2013-079371)
Patent document 4
Japanese translation of PCT International Application Publication No. 2006-523731 (WO2004-92223)
Patent document 5
Japanese translation of PCT International Application Publication No. 2007-521225 (WO2005-009408)
Patent document 6
International Publication WO2005-087289 (USP 7737214)
Patent document 7
International Publication WO2012-102210
Patent document 8: International Publication WO 2010/043979

SUMMARY OF THE INVENTION

PROBLEMS TO BE SOLVED BY THE INVENTION

The pharmaceutical compositions and the like of the patent documents 1 through 5 are incapable of achieving the disappearance of the gel and the release of the medication at the same time. The release amount of pharmaceutical compositions of the patent documents 2 and 3 is linear with respect to time. The pharmaceutical compositions of the patent documents 2 and 3 does not have a preferable profile of allowing the drug concentration to rise quickly and to be constant for a certain period of time. The solvents for use in the anesthetic agents disclosed in the patent documents 2, 3, and 5 are immiscible in water. Many of the solvents immiscible in water are toxic and undesirable for a part of a body underwent surgery of a body. The adhesion prevention agent of the patent document 6 is effective in that it remains present for a certain period time. But the adhesion prevention agent does not contain a medication and does not have a drug sustained release property.

It is an object of the invention to provide a sustained-release agent for topical administration in which the drug concentration in blood of a compound having a pharmacological action rises quickly after the sustained-release topically administered agent is administered to a part of a body and remains constant for a certain period of time, the compound is continuously released, and the release of the compound and the disappearance of a gel terminate at the same time. In the following, the expression "topically administered" has to be understood as being "for topical administration".

MEANS FOR SOLVING THE PROBLEMS

The sustained-release topically administered agent for achieving the above-described object is as described below.

The sustained-release topically administered agent of the invention is used after mixing. The sustained-release agent for topical administration is in the form of a kit to be mixed, said agent has a compound having a pharmacological action, a crosslinking polysaccharide derivative having at least one active ester group, introduced into a side chain of a polysaccharide, which is capable of reacting with an active hydrogen-containing group, and being capable of forming a crosslinked substance by a covalent bond between said active ester group and said active hydrogen-containing group; and a pH adjuster, not mixed with the crosslinking polysaccharide derivative, for adjusting a pH condition suitable for forming a biodegradable gel containing the compound when said sustained-release topically administered agent is mixed. The sustained-release topically administered agent forms the biodegradable gel containing the compound when said sustained-release topically administered agent is mixed. The pH of the biodegradable gel to be formed is not less than five.

BRIEF DESCRIPTION OF THE DRAWINGS

Fig. 1 is a graph showing the result of an experiment conducted by using the sustained-release topically administered agent of the invention.
Fig. 2 is a graph showing the result of the experiment conducted by using the sustained-release topically administered agent of the invention.

MODE FOR CARRYING OUT THE INVENTION

The sustained-release topically administered agent of the invention is described below by using embodiments. The invention is defined by the claims.

The sustained-release topically administered agent of the invention is used after mixing. The sustained-release topically administered agent has a compound having a pharmacological action, a crosslinking polysaccharide derivative having at least one active ester group, introduced into a side chain of a polysaccharide, which is capable of reacting with an active hydrogen-containing group, and being capable of forming a crosslinked substance by a covalent bond between the active ester group and the active hydrogen-containing group; and a pH adjuster, not mixed with the crosslinking polysaccharide derivative, for adjusting a pH condition suitable for forming a biodegradable gel containing the compound when said sustained-release topically administered agent is mixed. The sustained-release topically administered agent forms the biodegradable gel containing the compound when said sustained-release topically administered agent is mixed. The pH of the biodegradable gel to be formed is not less than five.

The crosslinking polysaccharide derivative for use in the sustained-release topically administered agent of the invention is described below.

The crosslinking polysaccharide derivative has at least one active ester group, introduced into the side chain of the polysaccharide, which is capable of reacting with the active hydrogen-containing group. The polysaccharide (raw material) into which the active ester group is introduced will be described later. Since a polysaccharide molecule substantially contains a hydroxyl group, namely, the active hydrogen-containing group, the polysaccharide derivative having the active ester group introduced into the polysaccharide has the active ester group and the active hydrogen-containing group in one molecular chain thereof and exhibits a self-crosslinking property under a reaction condition. The "self-crosslinking property" as referred to herein means that the active ester group and the active hydrogen-containing group react with each other in one molecule of the polysaccharide derivative or among the molecules thereof to form a covalent bond. In a case where the active hydrogen-containing group present on a biological surface is utilized for the reaction, the crosslinking polysaccharide derivative exhibits adhesiveness to the biological surface.

In this specification, the crosslinking polysaccharide derivative may be sometimes called an active esterified polysaccharide and may be sometimes hereinafter referred to as merely a polysaccharide derivative.

The molecule of "in one molecular chain" or "in one molecule" as referred to herein means one of molecules present in a range in which the molecules are continuously bound to one another by the covalent bond.

The polysaccharide derivative according to the invention is the active esterified polysaccharide and substantially holds a polysaccharide skeleton. Accordingly, the polysaccharide derivative may be sometimes hereinafter described in a manner parallel to a method of esterifying the polysaccharide (method of producing polysaccharide derivative).

In the invention, the active ester group to be introduced into the polysaccharide is essentially required to react with the active hydrogen-containing group to form the covalent bond. Such an active ester group is a group formed by a stronger electrophilic group than an ordinary ester is bonded to a carbonyl carbon of a carboxy group or a methyl carboxy group self-contained by the polysaccharide molecule or a carbonyl carbon of a carboxy group or a methylcarboxy group introduced into a polysaccharide by oxidation type introduction. When the active ester group is expressed by "-COOX", it is preferable that the electrophilic group which forms an alcohol site of "-OX" is a group introduced into the polysaccharide from an N-hydroxyamine-based compound. Because the N-hydroxyamine-based compound is a comparatively inexpensive raw material, it is industrially easy to perform an operation of introduce the active ester group into the polysaccharide.

Representative examples of the N-hydroxyamine-based compound to be used to form the above-described "-OX" include N-hydroxysuccinimide, N-hydroxynorbornene-2,3-dicarboxylic acid imide, ethyl 2-hydroxyimino-2-cyanoacetate, 2-hydroxyimino-2-cyanoacetic acid amide, and N-hydroxypiperidine.

In the invention, the active ester group of the polysaccharide derivative may be present therein singly or as an admixture of two or more kinds thereof.

Of these active ester groups, a succinimidyl ester group is preferable.

The polysaccharide derivative to be used in the invention contains at least one active ester group in the molecule thereof. In order to form a crosslinked matrix, the polysaccharide derivative usually contains two or more active ester groups in one molecule thereof. Though the amount of the polysaccharide derivative varies depending upon the purpose of use, it is preferable to set the amount thereof to 0.1 to 2 mmol/g in terms of the amount of the active ester group per gram of the dry weight of the polysaccharide derivative.

In the invention, the polysaccharide into which the active ester group is introduced and which constitutes a major skeleton of the polysaccharide derivative is not specifically limited in its structure so far as the polysaccharide contains two or more units of a monosaccharide structure in the major skeleton. Examples of such polysaccharides include monosaccharides such as arabinose, ribose, xylose, glucose, mannose, galactose, fructose, sorbose, rhamnose, fucose, and ribodesose; disaccharides such as trehalose, sucrose, maltose, cellobiose, gentiobiose, lactose, and melibiose; and trisaccharides or more polysaccharides such as raffinose, gentianose, melezitose, and stachyose. Polysaccharides is formed by the covalent bond of the above saccharides. Polysaccharides may have introduced functional groups. In the invention, polysaccharides may be present in nature or artificially synthesized. The polysaccharide derivative according to the invention can be made up of the skeleton of one kind of the polysaccharide or the skeletons of two or more kinds thereof.

The weight-average molecular weight of the polysaccharide forming the major skeleton of the polysaccharide derivative is not specifically limited. It is favorable that the polysaccharides have a weight-average molecular weight of 5,000 to 2,500,000 corresponding to polysaccharides formed by binding several tens to several thousands of the monosaccharides, the disaccharides, the trisaccharides or more polysaccharides to one another. This is because the polysaccharides having the above-described weight-average molecular weight allow the hardness of a gel formed after the polysaccharide derivative according to the invention is crosslinked to be readily adjusted and plural active ester groups and active hydrogen-containing groups to be readily introduced into one molecular chain. It is more favorable that the polysaccharide has a weight-average molecular weight of 10,000 to 1,000,000.

It is preferable that the polysaccharide (the polysaccharide may be sometimes hereinafter referred to as an acid group-containing polysaccharide) which forms the major skeleton of the polysaccharide derivative has the above-described structural component and a carboxylic acid group for forming the active ester group "--COOX" in a precursor stage of active esterification. The carboxylic acid group as referred to herein means a carboxy group and/or a carboxyalkyl group (these groups may be sometimes hereinafter referred to as the carboxylic acid group). The carboxyalkyl group means a functional group in which the carboxy group is bound to an alkyl skeleton as with a carboxymethyl group, the carboxyethyl group, a carboxypropyl group, a carboxyisopropyl group, a carboxybutyl group, and the like.

The above-described raw material polysaccharide is essentially required to be an acid group-containing polysaccharide at a precursor stage of the crosslinking polysaccharide derivative and thus may be a natural polysaccharide having the carboxylic acid group or a polysaccharide, not having the carboxylic acid group, into which the carboxy group and/or the carboxyalkyl group are introduced. Of these carboxylic acid group-containing polysaccharides, natural polysaccharides having the carboxy group, carboxylated polysaccharides having the carboxy group introduced thereinto, carboxymethylated polysaccharides having the carboxymethyl group introduced thereinto, and carboxyethylated polysaccharides having the carboxyethyl group introduced thereinto are favorable. The natural polysaccharides having the carboxy group, the carboxylated polysaccharides having the carboxy group introduced thereinto, and the carboxymethylated polysaccharides having the carboxymethyl group introduced thereinto are more favorable.

Though the above-described natural polysaccharides having the carboxylic acid group are not limited to specific ones, pectin containing galacturonic acid and hyaluronic acid are exemplified. For example, "GENUE pectin" of CP Kelco Inc. (Denmark) is exemplified as the pectin. "Hyaluronic Acid FCH" of Kibun Foods Inc. (Japan) is exemplified as the hyaluronic acid. It is possible to utilize commercially distributed products. The pectin is a polysaccharide containing the galacturonic acid as its main component. Approximately 75 to 80% of the pectin consists of the galacturonic acid. Other components of the pectin consist mainly of other saccharides. The pectin is the polysaccharide consisting of the galacturonic acid and other saccharides bound thereto in the above-described proportion. The hyaluronic acid is used as an auxiliary agent for an ophthalmic operation, a treatment agent for osteoarthritis of the knee, and so on. The hyaluronic acid does not contain galacturonic acid.

In the invention, it is desirable that the carboxy group and/or the carboxyalkyl group of the polysaccharide derivative are of a "non-salt type" not having a salt coordinated therewith and that the ultimately obtained polysaccharide derivative is not in a salt form. The "salt" as referred to herein includes inorganic salts such as the alkali metals, the alkaline earth metals, and the like, quaternary amines such as tetra-butylammonium (TBA), and halogen salts such as iodide chloromethyl pyridinium. The term of "non-salt type" as referred to herein means that any of the above-described "salts" is not coordinated with the carboxy group and/or the carboxyalkyl group. The term of "not in a salt form" means that the polysaccharide derivative does not contain any of the above-described salts.

Although the above-described polysaccharides into which the carboxy group and/or the carboxyalkyl group are introduced are not limited to specific ones, dextran and pullulan are exemplified as such polysaccharides.

The dextran is used as a plasma substitute. "Dextran T fractions" of Amersham Biosciences K.K. (Japan) is exemplified as the dextran. "Pullulan PI-20" of Hayashibara Biochemical Laboratories, Inc. (Japan) is exemplified as the pullulan. The pullulan is used as a medicinal additive including oral drugs. In particular, the pullulan having less biological contamination such as endotoxin is preferable.

In the invention, it is possible to utilize polysaccharides commercially distributed products. It is possible to suitably utilize the above-described polysaccharides which have brought actual performance in the medical applications from the standpoint of safety.

A carboxylation reaction of the polysaccharide can be carried out without particular limitation by utilizing a known oxidation reaction. Though the type of the carboxylation reaction is not specifically limited, dinitrogen tetroxide oxidation, fuming sulfuric acid oxidation, phosphoric acid oxidation, nitric acid oxidation, and hydrogen peroxide oxidation are exemplified. In the respective carboxylation reactions, using a reagent, the oxidation can be carried out by selecting a known reaction. The condition of each carboxylation reaction can be appropriately set up according to an introduction amount of the carboxy group. For example, it is possible to prepare a carboxylated polysaccharide by suspending the raw material polysaccharide in chloroform or carbon tetrachloride and adding dinitrogen tetroxide thereto so as to oxidize a hydroxyl group of the polysaccharide.

A carboxyalkylation reaction is not specifically limited. A known carboxyalkylation reaction of a polysaccharide can be utilized. In the case of a carboxymethylation reaction, it is possible to select a reaction in which monochloroacetic acid is used after a polysaccharide is alkalized. The condition of the reaction can be appropriately set up according to the introduction amount of a carboxymethyl group.

In the invention, as a method for introducing the carboxylic acid group into the polysaccharide, the above-described carboxylation or carboxyalkylation can be utilized without particular limitations. However, the carboxyalkylation, especially the carboxymethylation is more suitable in that the molecular weight of the polysaccharide is decreased to a lower extent by the carboxy group introduction reaction and that the introduction amount of the carboxy group is comparatively easily controlled.

In the invention, the introduction of the carboxylic acid group is not limited to a polysaccharide not having the carboxylic acid group. The carboxy group and/or the carboxymethyl group may be introduced into the natural polysaccharide, for example, the hyaluronic acid having the carboxylic acid group.

In the active esterification of the carboxy group and/or the carboxymethyl group of the above-described acid group-containing polysaccharide, the acid group-containing polysaccharide may be used singly or in combination of two or more kinds thereof.

The amount of the acid group-containing polysaccharide to be used in the active esterification is normally 0.1 to 5 mmol/g, favorably 0.4 to 3 mmol/g, and more favorably 0.6 to 2 mmol/g in terms of the amount of the carboxylic acid group (the carboxylic acid group is regarded as one molecule) per gram of the dry weight of the acid group-containing polysaccharide. When the amount of the carboxylic acid group is less than 0.1 mmol/g per gram of the dry weight of the acid group-containing polysaccharide, it often occurs that the number of the active ester groups which are derived from the carboxylic group and which become crosslinking points is insufficient. On the other hand, when the amount of the carboxylic acid group exceeds 5mmol/g per gram of the dry weight of the acid group-containing polysaccharide, the polysaccharide derivative (uncrosslinked) is sparingly soluble in a water-containing solvent.

A method of subjecting the acid group-containing polysaccharide to an active esterification method (production method of polysaccharide derivative) is not limited to a specific method. It is possible to exemplify a method of allowing the acid group-containing polysaccharide to react with an electrophilic group-introducing agent in the presence of a dehydration condensing agent and a method of using an ester exchange reaction for introducing the active ester group into the polysaccharide from an active ester group-containing compound. Of these methods, the former method is suitable in the invention. This method (the method of the invention) will be mainly described below. A method of forming a crosslinked polysaccharide is not claimed.

In carrying out the above-described preferred method of the invention, the acid group-containing polysaccharide is prepared in a solution containing an aprotic polar solvent to allow the acid group-containing polysaccharide to undergo a reaction. More concretely, the method includes a solution preparation step for dissolving a polysaccharide having the carboxy group or the carboxyalkyl group in the aprotic polar solvent, a reaction step for adding the electrophilic group-introducing agent and the dehydration condensing agent to the solution to subject the carboxy group of the polysaccharide or the carboxyalkyl group thereof to active esterification, a step of purifying a reaction product, and a step of drying the reaction product.

At the solution preparation step, dissolution of the polysaccharide in the aprotic polar solvent is achieved by adding the polysaccharide to the solvent and heating the solvent at 60 to 120 degrees C.

Accordingly, as the acid group-containing polysaccharide to be subjected to the active esterification in this method, among the above-enumerated polysaccharides, those which are dissolved in the aprotic polar solvent at a temperature of 60 to 120 degrees C are preferably used. Specifically, as the polysaccharide to be used in the reaction of introducing the electrophilic group into the carboxy group or the carboxyalkyl group, it is preferable that the carboxy group or the carboxymethyl group is of an acid type in view of the solubility of the polysaccharide in the aprotic polar solvent. The "acid type" as referred to herein means that a counter cation species of the carboxy group or that of the carboxymethyl group is a proton. A polysaccharide having the acid-type carboxy group is referred to as an acid-type (raw material) polysaccharide. For example, pectin which is a polysaccharide having the carboxy group is referred to as acid-type pectin. Carboxymethyl dextran having the acid-type carboxymethyl group is referred to as acid-type carboxymethyl (CM) dextran (acid-type CM dextran). In the "acid type", the counter cation species of the carboxy group and/or the carboxyalkyl group is a proton and thus, the acid type is not in a salt form. Therefore, the acid type is synonymous with the above-described "non-salt type".

The "aprotic polar solvent" as referred to herein means a polar solvent which does not have a proton capable of forming a hydrogen bond with a nucleophilic agent having an electrically positive functional group. Although the aprotic polar solvent which can be used in the production method according to the invention is not limited to a specific one, it is possible to exemplify dimethyl sulfoxide (DMSO), N,N-dimethylformamide, N-methyl-2-pyrrolidone, N,N-dimethylacetamide, and 1,3-dimethyl-2-imidazolidinone. The dimethyl sulfoxide can be suitably utilized because it allows the polysaccharide to have a high solubility in a solvent.

At the reaction step, the carboxy group of the polysaccharide and/or the carboxymethyl group thereof are subjected to the active esterification by adding the electrophilic group-introducing agent and the dehydration condensing agent to an acid-type polysaccharide solution. Although a reaction temperature at the time of the active esterification is not specifically limited, the reaction temperature is favorably 0 to 70 degrees C and more favorably 20 to 40 degrees C. Although a reaction period of time varies with the reaction temperature, the reaction period of time is set to normally 1 to 48 hours and favorably 12 to 24 hours.

The "electrophilic group-introducing agent" as referred to herein means a reagent for introducing the electrophilic group into the carboxy group or carboxyalkyl group and changing the carboxy group or the carboxyalkyl group into the active ester group. Although the kind of the electrophilic group-introducing agent is not limited to a specific one, it is possible to utilize an active ester-inducing compound widely used to synthesize peptide. As the active ester-inducing compound, N-hydroxyamine based active ester-inducing compounds are exemplified. Although the N-hydroxyamine based active ester-inducing compound is not limited to specific ones, N-hydroxysuccinimide, N-hydroxynorbornene-2,3-dicarboxylic acid imide, ethyl 2-hydroxyimino-2-cyanoacetate, 2-hydroxyimino-2-cyanoacetic acid amide, and N-hydroxypiperidine are exemplified. Of these N-hydroxyamine based active ester-inducing compounds, the N-hydroxysuccinimide is suitable because it has brought actual performance in the field of the synthesis of the peptide and is readily commercially available.

In changing the carboxy group or the carboxyalkyl group into the active ester group by using the electrophilic group-introducing agent, the "dehydration condensing agent" draws one water molecule generated by the condensation between the carboxy group or the carboxyalkyl group and the electrophilic group-introducing agent, i.e., carries out dehydration, thereby subjecting the carboxy group or the carboxyalkyl group and the electrophilic group to an ester bond. Although the kind of the dehydration condensing agent is not limited to a specific one, it is possible to exemplify 1-ethyl-(3-dimethylaminopropyl)carbodiimide hydrochloride (EDC ∙ HCl) and 1-cyclohexyl-(2-morphonyl-4-ethyl)-carbodiimide ∙ meso-p-toluenesulfonate. Of these dehydration condensing agent, the 1-ethyl-(3-dimethylaminopropyl)carbodiimide hydrochloride (EDC ∙ HCl) is suitable because it has brought actual performance in the peptide synthesis field and is readily commercially available.

At the purification step, after the reaction step finishes, the unreacted electrophilic group-introducing agent, the dehydration condensing agent, and reaction by-products are removed from the reaction solution by means of reprecipitation, filtration and/or cleaning normally used. Thereby the polysaccharide derivative according to the invention can be obtained.

At the drying step, to remove the cleaning solvent from the polysaccharide derivative obtained at the purification step, the polysaccharide derivative is dried by a method normally used.

In the invention, as described previously, it is preferable that the final amount of the polysaccharide derivative is 0.1 to 2 mmol/g in terms of the amount of the active ester group per gram of the dry weight of the polysaccharide derivative. In the above, to obtain such a polysaccharide derivative, it is possible to control the amount of the active ester group to be introduced into the carboxy group of the active esterified raw material polysaccharide.

In order to control the introduction amount of the active ester group, it is possible to adjust the mixing amount of the electrophilic group-introducing agent and that of the dehydration condensing agent at the above-described reaction step. More specifically, it is preferable that the ratio (Z/X) of the molar number (Z mmol) of the dehydration condensing agent to the molar number (X mmol) of all carboxy groups of the polysaccharide is set to 0.1<Z/X<50 to satisfy the condition regarding the addition amount of the dehydration condensing agent at the above-described reaction temperature. This is because when the ratio of Z/X is less than 0.1, the addition amount of the dehydration condensing agent is small. Thus, the reaction efficiency is low, which makes it difficult to achieve a desired rate of introduction of the active ester group. When the ratio of Z/X is more than 50, the addition amount of the dehydration condensing agent is large. Thus, though the rate of introduction of the active ester group is high, the resulting polysaccharide derivative is sparingly soluble in water.

The ratio of the molar number (Y mmol) of the electrophilic group-introducing agent to the molar number (X mmol) of the total carboxy groups of the polysaccharide is not limited to a specific ratio so far as the electrophilic group-introducing agent is added in an amount not less than a reaction amount according to the rate of introduction of the active ester group. It is preferable that the ratio of Y/X is set to 0.1<Y/X<100 to satisfy the condition regarding the reaction amount of the electrophilic group-introducing agent.

The polysaccharide derivative according to the invention has the hydroxyl group of a glucopyranose ring in the polysaccharide skeleton molecule after the active ester group is introduced into the carboxy group of the polysaccharide and thus, has the active hydrogen-containing group. The active hydrogen-containing group in the molecule is not limited thereto. The polysaccharide derivative may further have the active hydrogen-containing group introduced into the molecule thereof as necessary. In this case, the polysaccharide derivative may have one kind or two or more kinds of the active hydrogen-containing group.

The polysaccharide derivative according to the invention is capable of containing known elements and functional groups such as atomic groups in addition to the active ester group and the active hydrogen-containing group so far as the characteristics of the invention are not impaired. Specific examples of the elements and the functional group include halogen elements such as fluorine, chlorine, bromine, and iodine; the carboxy group; carboxyalkyl groups such as a carboxymethyl group, a carboxyethyl group, a carboxypropyl group, and a carboxyisopropyl group; a silyl group; an alkylenesilyl group; an alkoxysilyl group; and a phosphoric acid group. Such a functional group may be introduced singly or in admixture of two or more kinds thereof.

The rate (%) of introduction of the active ester group can be expressed by multiplying 100 by a ratio (AE/TC) of the molar number (AE) of the content of the active ester group in the resulting polysaccharide derivative to the molar number of the content of the carboxy group and the molar number of the content of the carboxymethyl group which the active esterified raw material polysaccharide has (hereinafter expressed as the total carboxy groups (TC)).

The rate of introduction of the active group can be determined by, for example, a method described in Biochemistry Vol. 14, No. 7 (1975), pages from 1535 to 1541.

The polysaccharide derivative may have the carboxy group and/or the carboxymethyl group which remain in the raw material polysaccharide in a case where the active ester group is introduced into the raw material polysaccharide at an introduction rate of less than 100%.

The "crosslinking structure" as referred to herein means that a covalent bond is formed in a single molecular chain of the polysaccharide derivative according to the invention and/or among plural molecular chains thereof and as a result, the molecular chains of the polysaccharide derivative take a reticular three-dimensional structure. In this crosslinking, the active ester group and the active hydrogen-containing group can be bound to each other in one molecular chain. In addition, a plurality of molecules may be crosslinked to one another by the covalent bond. The polysaccharide derivative according to the invention soluble in water before undergoing a crosslinking-forming reaction forms the crosslinking structure with the progress of the reaction. As a result, the polysaccharide derivative decreases its flowability and becomes a water-insoluble lump (hydrogel) to form a polysaccharide crosslinked polymer. Defining the property of the polysaccharide derivative capable of forming the crosslinking structure by the covalent bond in its one molecular chain or among its molecular chains without using other crosslinking agents as "self-crosslinking property", the saccharide derivative according to the invention is a self-crosslinking polysaccharide.

The polysaccharide derivative according to the invention has the self-crosslinking property owing to the involvement of the intramolecular active hydrogen-containing group as described previously. In addition, by applying the polysaccharide derivative to the biological surface, the polysaccharide derivative is able to exhibit the adhesiveness to the biological surface owing to the reaction which occurs between the active hydrogen-containing group and the active ester group. Such a form of the use of the polysaccharide derivative according to the invention is a preferable embodiment thereof. When the polysaccharide derivative is applied to the biological surface, the polysaccharide derivative may display the self-crosslinking property.

In the invention, the active hydrogen-containing group which is involved in the reaction between the active hydrogen-containing group and the active ester group is not specifically limited so far as the active hydrogen-containing group is capable of forming the covalent bond by the reaction between the active hydrogen-containing group and the active ester group under a reaction condition specified in the invention. In the invention, it is possible to use active hydrogen-containing groups normally used. Specific examples of the active hydrogen-containing group include a hydroxyl group, an amino group, and a thiol group. The above-described amino group includes a primary amino group and a secondary amino group. Above all, the hydroxyl group and the primary amino group are preferable as the active hydrogen-containing group because the reactivity thereof with the active ester group is good and in addition, the hydroxyl group and the primary amino group allow the polysaccharide derivative to be crosslinked and the biodegradable gel to be formed in a short period of time.

In the invention, the above-described crosslinking reaction of the polysaccharide derivative is based on the formation of the covalent bond caused by the reaction between the active ester group and the active hydrogen-containing group. Specifically, a method of crosslinking the polysaccharide derivative by adding a pH adjuster to a solution of the polysaccharide derivative is exemplified.

In the crosslinking polysaccharide derivative of the invention, because heat does not substantially make a great contribution to the crosslinking reaction, reaction temperature is not specifically limited. It is possible to set the reaction temperature to a range of 10 to 40 degrees C.

The polysaccharide derivative can be used in a powder form or a sheet-like form. That is, the powder polysaccharide derivative can be obtained by crushing or pulverizing the polysaccharide derivative obtained by the above-described synthesis reaction and adjusting the particle size to an appropriate range as necessary. In order to make the particle size small, the polysaccharide derivative is subjected to freezing and pulverizing, mill crushing and/or classification, although the particle size-decreasing means is not limited to a specific one. After the polysaccharide derivative is crushed or pulverized and sieved, resulting powder can be adjusted to an arbitrary particle size distribution. Although an average particle size is not specifically limited, it is preferable to set the average particle size to several tens of nanometers to several hundreds of micrometers. The resulting powders can be prepared into a paste-like form or aerosols by a method normally used.

The sheet-like polysaccharide derivative can be produced by carrying out a solution preparation step for dissolving the polysaccharide derivative in water and a drying step for heat drying or freeze drying the solution after spreading the solution in a desired configuration. More specifically, the sheet-like polysaccharide derivative can be obtained by preparing an aqueous solution in which the polysaccharide derivative has been dissolved and freeze-drying the aqueous solution. In preparing the sheet-like polysaccharide derivative, the pH of water for preparing the aqueous solution is preferably 3.0 to 7.5. This is because when the pH of water is less than 3.0, the resulting sheet exhibits a strong acidity, whereas when the pH thereof is more than 7.5, the active ester group may possibly liberate. The heat-dried sheet can be obtained by spreading the aqueous solution on a base material and heat-drying it at 30 to 110 degrees C. The aqueous solution can be heat-dried under a decreased pressure as necessary. The freeze-dried sheet can be obtained by freezing and drying the aqueous solution. It is possible to use an ordinary freeze dryer.

Though the timing of mixing a pH adjuster (B) and a polysaccharide derivative (A) with each other is not specifically limited, the mixing timing is appropriately selected before use and during use. A composition containing the polysaccharide derivative (A) and the pH adjuster (B) may contain other substances as necessary. The other substances may be mixed with the polysaccharide derivative or may not be mixed therewith.

The pH adjuster (B) to be used in the invention means an aqueous solution, a water-containing solvent or a salt (powder) etc. for adjusting the pH of the biodegradable gel according to the invention to 5.0 to 14 and preferably to 6.4 to 12. The kind of the pH adjuster (B) is not limited to a specific one. Examples thereof include a sodium carbonate aqueous solution or powder, sodium hydrogen carbonate aqueous solution or powder, a phosphoric acid-based buffer agent (disodium hydrogen phosphate-dipotassium hydrogen phosphate), and an acetic acid-ammonia based buffer agent. Above all, the sodium hydrogen carbonate can be suitably used as a pH adjuster for medical use in such a way that a 7% aqueous solution (pH 8.3) thereof is utilized as an intravenous injection solution. Thus, the sodium hydrogen carbonate can be preferably used in terms of safety.

Examples of the form of the above-described composition containing the polysaccharide derivative (A) and the pH adjuster (B) include a two-component system consisting of an aqueous solution in which the concentration of the polysaccharide derivative is 1 to 80% (w/v) and water, having a pH 7.5 to 10.5, which is held separately from the aqueous solution.

In this system, by mixing the aqueous solution and the water with each other at the time of use, it is possible to prepare a mixed aqueous solution in which the final concentration of the polysaccharide derivative is 0.1 to 60% (w/v). It is also possible to exemplify a mixed aqueous solution prepared at the time of use by adding a salt of the pH adjuster (B) to the aqueous solution of the polysaccharide derivative having a concentration of 1 to 80% (w/v) and by mixing the salt of the pH adjuster while dissolving the salt of the pH adjuster in the aqueous solution so that the final concentration of the polysaccharide derivative is 0.1 to 80% (w/v). Although a normal mixing method can be selected, it is preferable to mix the salt of the pH adjuster and the polysaccharide derivative with each other until both are uniformly mixed with each other to such an extent that a desired reaction proceeds.

In the invention, it is possible to use a crosslinking polysaccharide composition containing the polysaccharide derivative (A) and a polymer (C). The polymer (C) is used to adjust the hardness and property of a hydrogel when the polysaccharide composition is crosslinked. The polysaccharide composition may contain one kind of the polysaccharide derivative (A) or two or more kinds thereof.

Though the kind of the polymer (C) is not specifically limited, it is preferable to use the polymer (C) containing two or more primary amino groups, thiol groups or hydroxyl groups in one molecule thereof. Specific examples of the polymer (C) include polyalkylene glycol derivatives, polypeptides, and polysaccharides or derivatives thereof. Though the content of the polymer (C) contained in the polysaccharide composition according to the invention is not specifically limited, it is preferable that the polymer (C) is contained at 5% to 50% by mass for the entire polysaccharide composition. The polymer (C) can be used singly or in combination of two or more kinds thereof.

Examples of the above-described polyalkylene glycol derivatives include polyethylene glycol (PEG) derivatives, polypropylene glycol derivatives, polybutylene glycol derivatives, and polypropylene glycol-polyethylene glycol block copolymer derivatives, and random copolymer derivatives. Examples of the basic polymer skeletons of the polyethylene glycol derivatives include ethylene glycol, diglycerol, pentaerythritol, and hexaglycerol. The molecular weights of the polyalkylene glycol derivatives are favorably 100 to 50,000 and more favorably 1,000 to 20,000.

The kind of above-described polyethylene glycol derivatives is not specifically limited. Examples thereof include an ethylene glycol type polyethylene glycol derivative containing a thiol group at both ends thereof and having a weight-average molecular weight of 1,000, 2,000, 6,000 or 10,000; an ethylene glycol type polyethylene glycol derivative containing an amino group at both ends thereof and having a weight-average molecular weight of 1,000, 2,000, 6,000 or 10,000; a trimethylolethane type polyethylene glycol derivative containing the thiol group at three ends thereof and having a weight-average molecular weight of 5,000 or 10,000; a trimethylolethane type polyethylene glycol derivatives containing the amino group at three ends thereof and having a weight-average molecular weight of 5,000 or 10,000; a diglycerol type polyethylene glycol derivative containing the thiol group at four ends thereof and having a weight-average molecular weight of 5,000, 10,000 or 20,000; a diglycerol type polyethylene glycol derivative containing the amino group at four ends thereof and having a weight-average molecular weight of 5,000, 10,000 or 20,000; a pentaerythritol type polyethylene glycol derivative having the thiol group at four ends thereof and having a weight-average molecular weight of 10,000 or 20,000; a pentaerythritol type polyethylene glycol derivative having the amino group at four ends thereof and having a weight-average molecular weight of 10,000 or 20,000; a hexaglycerol type polyethylene glycol derivative containing the thiol group at eight ends thereof and having a weight-average molecular weight of 10,000 or 20,000; and a hexaglycerol type polyethylene glycol derivative containing the amino group at eight ends thereof and having a weight-average molecular weight of 10,000 or 20,000.

The "weight-average molecular weight" as referred to herein is one of numeral values expressing the average molecular weight of a polymer. The polymer is the mixture of molecules having the same basic structural unit and different molecular lengths (chain lengths). Therefore, the polymer has a molecular weight distribution according to the difference in the molecular chain length. In order to express the molecular weight, the average molecular weight is used. Although the average molecular weight includes the weight-average molecular weight and the number average molecular weight, the weight-average molecular weight is used herein. In the invention, the value (100%) of the weight-average molecular weight includes a case where the upper limit thereof is 110% and a case where the lower limit is 90% with respect to 100%. The polyethylene glycol derivative can be prepared in accordance with, for example, the method described in Chapter 22 of Poly(ethylene Glycol) Chemistry: Biotechnical and Biomedical Applications edited by J. Milton Harris, Plenum Press, NY (1992). The polyethylene glycol derivative can be chemically modified in such a way that the polyethylene glycol contains one or plural primary amino groups or thiol groups. It is possible to purchase the polyethylene glycol derivative (SUNBRIGHT HGEO-20TEA and SUNBRIGHT PTE-10TSH) produced by NOF CORPORATION.

Although the kind of the above-described polypeptides is not specifically limited, it is possible to exemplify collagen, gelatin, albumin, and polylysine. Although the kind of the polysaccharide is not specifically limited, it is possible to exemplify pectin, hyaluronic acid, chitin, chitosan, carboxymethyl chitin, carboxymethyl chitosan, chondroitin sulfate, keratin sulfate, keratosulfate, heparin, and derivatives thereof.

In the polysaccharide composition containing the polysaccharide derivative (A) and the polymer (C), suitable combinations of the polysaccharide derivative (active esterified polysaccharide) (A) and the polymer (C) are as described below. In these combinations, the configuration (sheet-like form, powder form, and liquid form) of the polysaccharide composition can be appropriately selected with reference to the examples described later.

A combination of active esterified pectin and at least one polymer (C) selected from among a group consisting of an ethylene glycol type PEG derivative containing a thiol group at both ends thereof, an ethylene glycol type PEG derivative containing an amino group at both ends thereof, a trimethylolethane type PEG derivative containing the thiol group at three ends thereof, a trimethylolethane type PEG derivative containing the amino group at three ends thereof, a pentaerythritol type PEG derivative containing the thiol group at four ends thereof, a pentaerythritol type PEG derivative containing the amino group at four ends thereof, a hexaglycerol type PEG derivative containing the thiol group at eight ends thereof, a hexaglycerol type PEG derivative containing the amino group at eight ends thereof, albumin, gelatin, collagen, polylysine, pectin, chitosan, chitin, and carboxymethy(CM) chitin.

A combination of active esterified CM dextran and at least one polymer (C) selected from among the group consisting of the ethylene glycol type PEG derivative containing the thiol group at both ends thereof, the ethylene glycol type PEG derivative containing the amino group at both ends thereof, the trimethylolethane type PEG derivative containing the thiol group at three ends thereof, the trimethylolethane type PEG derivative containing the amino group at three ends thereof, the pentaerythritol type PEG derivative containing the thiol group at four ends thereof, the pentaerythritol type PEG derivative containing the amino group at four ends thereof, the hexaglycerol type PEG derivative containing the thiol group at eight ends thereof, the hexaglycerol type PEG derivative containing the amino group at eight ends thereof, albumin, gelatin, collagen, polylysine, pectin, chitosan, chitin, and CM chitin.

A combination of active esterified CM pullulan and at least one polymer (C) selected from among the group consisting of the ethylene glycol type PEG derivative containing the thiol group at both ends thereof, the ethylene glycol type PEG derivative containing the amino group at both ends thereof, the trimethylolethane type PEG derivative containing the thiol group at three ends thereof, the trimethylolethane type PEG derivative containing the amino group at three ends thereof, the pentaerythritol type PEG derivative containing the thiol group at four ends thereof, the pentaerythritol type PEG derivative containing the amino group at four ends thereof, the hexaglycerol type PEG derivative containing the thiol group at eight ends thereof, the hexaglycerol type PEG derivative containing the amino group at eight ends thereof, albumin, gelatin, collagen, polylysine, pectin, chitosan, chitin, and the CM chitin.

A combination of active esterified CM hydroxyethyl starch and at least one polymer (C) selected from among the group consisting of the ethylene glycol type PEG derivative containing the thiol group at both ends thereof, the ethylene glycol type PEG derivative containing the amino group at both ends thereof, the trimethylolethane type PEG derivative containing the thiol group at three ends thereof, the trimethylolethane type PEG derivative containing the amino group at three ends thereof, the pentaerythritol type PEG derivative containing the thiol group at four ends thereof, the pentaerythritol type PEG derivative containing the amino group at four ends thereof, the hexaglycerol type PEG derivative containing the thiol group at eight ends thereof, the hexaglycerol type PEG derivative containing the amino group at eight ends thereof, albumin, gelatin, collagen, polylysine, pectin, chitosan, chitin, and the CM chitin.

A combination of active esterified pectin and at least one polymer (C) selected from among the group consisting of the ethylene glycol type PEG derivative containing the thiol group at both ends thereof and having the weight-average molecular weight of 1,000, 2,000, 6,000 or 10,000, the ethylene glycol type PEG derivative containing the amino group at both ends thereof and having the weight-average molecular weight of 1,000, 2,000, 6,000 or 10,000, the trimethylolethane type PEG derivative containing the thiol group at three ends thereof and having the weight-average molecular weight of 5,000 or 10,000, the trimethylolethane type PEG derivative containing the amino group at three ends thereof and having the weight-average molecular weight of 5,000 or 10,000, the diglycerol type PEG derivative containing the thiol group at four ends thereof and having the weight-average molecular weight of 5,000, 10,000 or 20,000, the diglycerol type PEG derivative containing the amino group at four ends thereof and having the weight-average molecular weight of 5,000, 10,000 or 20,000, the pentaerythritol type PEG derivative having the thiol group at four ends thereof and having the weight-average molecular weight of 10,000 or 20,000, the pentaerythritol type PEG derivative having the amino group at four ends thereof and having the weight-average molecular weight of 10,000 or 20,000, the hexaglycerol type PEG derivative containing the thiol group at eight ends thereof and having the weight-average molecular weight of 10,000 or 20,000, and the hexaglycerol type PEG derivative containing the amino group at eight ends thereof and having the weight-average molecular weight of 10,000 or 20,000.

A combination of active esterified CM dextran and at least one polymer (C) selected from among the group consisting of the ethylene glycol type PEG derivative containing the thiol group at both ends thereof and having the weight-average molecular weight of 1,000, 2,000, 6,000 or 10,000, the ethylene glycol type PEG derivative containing the amino group at both ends thereof and having the weight-average molecular weight of 1,000, 2,000, 6,000 or 10,000, the trimethylolethane type PEG derivative containing the thiol group at three ends thereof and having the weight-average molecular weight of 5,000 or 10,000, the trimethylolethane type PEG derivative containing the amino group at three ends thereof and having the weight-average molecular weight of 5,000 or 10,000, the diglycerol type PEG derivative containing the thiol group at four ends thereof and having the weight-average molecular weight of 5,000, 10,000 or 20,000, the diglycerol type PEG derivative containing the amino group at four ends thereof and having the weight-average molecular weight of 5,000, 10,000 or 20,000, the pentaerythritol type PEG derivative having the thiol group at four ends thereof and having the weight-average molecular weight of 10,000 or 20,000, the pentaerythritol type PEG derivative having the amino group at four ends thereof and having the weight-average molecular weight of 10,000 or 20,000, the hexaglycerol type PEG derivative containing the thiol group at eight ends thereof and having the weight-average molecular weight of 10,000 or 20,000, and the hexaglycerol type PEG derivative containing the amino group at eight ends thereof and having the weight-average molecular weight of 10,000 or 20,000.

A combination of active esterified CM pullulan and at least one polymer (C) selected from among the group consisting of the ethylene glycol type PEG derivative containing the thiol group at both ends thereof and having the weight-average molecular weight of 1,000, 2,000, 6,000 or 10,000, the ethylene glycol type PEG derivative containing the amino group at both ends thereof and having the weight-average molecular weight of 1,000, 2,000, 6,000 or 10,000, the trimethylolethane type PEG derivative containing the thiol group at three ends thereof and having the weight-average molecular weight of 5,000 or 10,000, the trimethylolethane type PEG derivative containing the amino group at three ends thereof and having the weight-average molecular weight of 5,000 or 10,000, the diglycerol type PEG derivative containing the thiol group at four ends thereof and having the weight-average molecular weight of 5,000, 10,000 or 20,000, the diglycerol type PEG derivative containing the amino group at four ends thereof and having the weight-average molecular weight of 5,000, 10,000 or 20,000, the pentaerythritol type PEG derivative having the thiol group at four ends thereof and having the weight-average molecular weight of 10,000 or 20,000, the pentaerythritol type PEG derivative having the amino group at four ends thereof and having the weight-average molecular weight of 10,000 or 20,000, the hexaglycerol type PEG derivative containing the thiol group at eight ends thereof and having the weight-average molecular weight of 10,000 or 20,000, and the hexaglycerol type PEG derivative containing the amino group at eight ends thereof and having the weight-average molecular weight of 10,000 or 20,000.

A combination of active esterified CM hydroxyethyl starch and at least one polymer (C) selected from among the group consisting of the ethylene glycol type PEG derivative containing the thiol group at both ends thereof and having the weight-average molecular weight of 1,000, 2,000, 6,000 or 10,000, the ethylene glycol type PEG derivative containing the amino group at both ends thereof and having the weight-average molecular weight of 1,000, 2,000, 6,000 or 10,000, the trimethylolethane type PEG derivative containing the thiol group at three ends thereof and having the weight-average molecular weight of 5,000 or 10,000, the trimethylolethane type PEG derivative containing the amino group at three ends thereof and having the weight-average molecular weight of 5,000 or 10,000, the diglycerol type PEG derivative containing the thiol group at four ends thereof and having the weight-average molecular weight of 5,000, 10,000 or 20,000, the diglycerol type PEG derivative containing the amino group at four ends thereof and having the weight-average molecular weight of 5,000, 10,000 or 20,000, the pentaerythritol type PEG derivative having the thiol group at four ends thereof and having the weight-average molecular weight of 10,000 or 20,000, the pentaerythritol type PEG derivative having the amino group at four ends thereof and having the weight-average molecular weight of 10,000 or 20,000, the hexaglycerol type PEG derivative containing the thiol group at eight ends thereof and having the weight-average molecular weight of 10,000 or 20,000, and the hexaglycerol type PEG derivative containing the amino group at eight ends thereof and having the weight-average molecular weight of 10,000 or 20,000.

A mixing ratio (SD/AP) of the polysaccharide derivative (SD) (A) to the polymer (C) (AP) is preferably 20/80 to 98/2 (w/w). This is because when the polymer (C) is mixed with the polysaccharide derivative (SD) in an amount more than 80% by mass, it is difficult to obtain the self-crosslinking property of the polysaccharide (A) due to the hindrance of the polymer (C). On the other hand, when the polymer (C) is mixed therewith in an amount less than 2% by mass, it is difficult to adjust the hardness and property of the hydrogel to be finally obtained.

Although the kind of the compound having the pharmacological action is not limited to a specific one, it is preferable to use at least one selected from among a group consisting of a local anesthetic medication, antibiotics, antimicrobial agents, antifungal agents, antiviral agents, anti-inflammatory agents, vasoconstricting agents, steroid hormone agent, antihistamines, prostaglandin, and anti-cancer drugs.

As the local anesthetic medication, it is preferable to use at least one selected from among a group consisting of ropivacaine, bupivacaine, levobupivacaine, lidocaine, procaine, tetracaine, cocaine, benzocaine, mepivacaine, prilocaine, dibucaine, chloroprocaine, and etidocaine or salts of these local anesthetic medications. In consideration of the visibility of the biodegradable gel to be formed, dibucaine hydrochloride, tetracaine hydrochloride, bupivacaine hydrochloride hydrate, procaine hydrochloride, mepivacaine hydrochloride, lidocaine hydrochloride, levobupivacaine hydrochloride, ropivacaine hydrochloride, etidocaine hydrochloride, prilocaine hydrochloride, and chloroprocaine hydrochloride are preferable.

Examples of the antibiotics include βlactam such as ampicillin, aminoglycosides such as kanamycin sulfate, tetracyclines such as tetracycline hydrochloride and minocycline, polypeptides such as polymyxin B sulfate, macrolides such as clarithromycin, and chloramphenicol. Examples of the antimicrobial agents include new quinolones such as tosufloxacin tosylate and synthetic antibacterial agents such as sulfamethoxazole trimethoprim combination. Examples of the antifungal agents include polyene macrolide such as amphotericin and azole. Examples of the antiviral agents include acyclovir, vidarabine, and the like. Examples of the anti-inflammatory agents include diclofenac, celecoxib, glycyrrhizin dipotassium, and lysozyme chloride. Examples of the vasoconstricting agents include naphazoline chloride, dl-methylephedrine hydrochloride, and the like. Examples of the steroid hormone agent include hydrocortisone butylate and the like. Examples of the antihistamines include diphenhydramine hydrochloride, chlorpheniramine maleate, and the like. Examples of the prostaglandin include dinoprost, dinoprostone, and the like.

Examples of the anti-cancer drugs include doxorubicin hydrochloride, peplomycin hydrochloride, nitrogen mustard-N-oxide hydrochloride, cyclophosphamide, thiotepa, carboquone, nimustine hydrochloride, bleomycin hydrochloride, bleomycin sulfate, peplomycin sulfate, aclarubicin hydrochloride, idarubicin hydrochloride, epirubicin hydrochloride, daunorubicin hydrochloride, pirarubicin hydrochloride, zinostatin stimalamer, neocarzinostatin, etoposide, teniposide, irinotecan hydrochloride, vincristine sulfate, vindesine sulfate, vinblastine sulfate, L-asparaginase, mitoxantrone hydrochloride, cisplatin, carboplatin, nedaplatin, pentostatin, schizophyllan, porfimer sodium, ifosfamide, katarubajin, mercaptopurine, thioinosine, cytarabine, enocitabine, fluorouracil, tegafur, ancitabine hydrochloride, methotrexate, carmofur, mitomycin C, actinomycin, bleomycin hydrochloride, and taxol.

It is preferable that the sustained-release topically administered agent of the invention has visibility to be brought about by a phenomenon that the biodegradable gel is suspended when the sustained-release topically administered agent is mixed. The visibility allows a part where the topically administered agent has been administered to be easily confirmed and the topically administered agent to be securely administered to a necessary part. It is conceivable that the visibility appears by the phenomenon that the biodegradable gel becomes clouded white when the sustained-release topically administered agent.

As the compound having the pharmacological action, it is preferable that the biodegradable gel formed becomes suspended, i.e., the biodegradable gel becomes cloudy white to such an extent that it can be distinguished from a biological tissue (pink to red). It is preferable to use the compound having the pharmacological action which becomes cloudy at a predetermined pH value. It is preferable that sustained-release topical administered agent is prepared so as to have the above-mentioned predetermined pH at the time of mixing.

In the sustained-release topically administered agent, the pH of the biodegradable gel to be formed when the sustained-release topically administered agent is mixed with each other is favorably 6.4 to 12 and especially favorably 7.5 to 10.5. The hardness of the biodegradable gel having a temperature of 20 degrees C is favorably 2500 to 20000N/m$^2$. To measure the hardness of the biodegradable gel, by placing it in a cylindrical container having a diameter of 40mm and a height of 15mm, a compression measurement was conducted at a compression speed of 600mm/minute and a clearance of 5mm by using a creep meter MODEL RE2-33005B (produced by Yamaden Engineering Inc.) and a plunger, made of resin, which has a diameter of 20mm and a height of 8mm. It is especially favorable that the hardness of the gel is 6000 to 6500N/m$^2$.

It is preferable that in the sustained-release topically administered agent, the release rate of the compound having the pharmacological action is not less than 8% during 1 hour from a formation of the biodegradable gel, the release rate of the compound having the pharmacological action is not more than 85% during 6 hours from the formation of the biodegradable gel.

The sustained-release property of the compound having the pharmacological action contained in the sustained-release topically administered agent of the invention varies with the kind thereof. In a case where the compound having the pharmacological action contained in the sustained-release topically administered agent is a local anesthetic agent, it is preferable that the release rate of the local anesthetic medication is not less than 8% from the time when the sustained-release topically administered agent is mixed (after a mixed sustained-release topically administered agent is administered) until 1 hour lapsed and is not more than 85% from the time when the sustained-release topically administered agent is mixed (after the mixed sustained-release topically administered agent is administered) until 6 hours lapsed. In a case where the release rate falls within the above-described range, the analgesic effect is displayed at an early stage from the time when the gelatinous local anesthetic medication is administered until before the lapse of one hour and in addition, the analgesic effect continues after the lapse of six hours. The release rate (%) is calculated by using [(amount of local anesthetic released by topically administered topically administered agent/total amount of local anesthetic medication in topically administered agent) $\times$ 100]. It is preferable that the release rate of the local anesthetic medication is not less than 80% after the lapse of 72 hours from the time when the local anesthetic medication is administered.

It is especially preferable that the release rate of the local anesthetic medication is not less than 10% during 1 hour from the time when the local anesthetic medication is administered and is less than 70% during 6 hours from the time when the local anesthetic medication is administered. It is especially preferable that the release rate of the local anesthetic medication is not less than 82% during 72 hours from the time when the local anesthetic medication is administered.

The in-vivo decomposition speed of the biodegradable gel formed by the sustained-release topically administered agent of the invention varies with a compound having a pharmacological action to be used. It is preferable that the decomposition rate of the biodegradable gel is not less than 8% from the time when the sustained-release topically administered agent is mixed (after the mixed sustained-release topically administered agent is administered) until before the lapse of one hour and is not more than 85% from the time when the sustained-release topically administered agent is mixed (after the mixed sustained-release topically administered agent is administered) until before the lapse of six hours. In a case where the decomposition rate of the sustained-release topically administered agent falls within the above-described range, the pharmacological effect is displayed at an early stage from the time when the topically administered agent is administered until before the lapse of one hour and in addition, the pharmacological effect continues after the lapse of six hours. It is preferable that the decomposition rate of the sustained-release topically administered agent is not less than 80% after the lapse of 72 hours from the time when the sustained-release topically administered agent is administered. It is especially preferable that the decomposition rate of the sustained-release topically administered agent is not less than 10% from the time when the sustained-release topically administered agent is administered until before the lapse of one hour and is less than 70% from the time when the sustained-release topically administered agent is administered until before the lapse of six hours. It is especially preferable that the decomposition rate of the sustained-release topically administered agent is not less than 82% from the time when it is administered until before the lapse of 72 hours.

The sustained-release topically administered agent of the invention is used after mixing. So long as the sustained-release topically administered agent contains the compound having the pharmacological action, the crosslinking polysaccharide derivative, and the pH adjuster, the sustained-release topically administered agent may have any dosage form. The crosslinking polysaccharide derivative in the form of powder is more stable than in the form of liquid in its gel-forming property.

As the dosage form of the sustained-release topically administered agent, conceivably, the sustained-release topically administered agent is composed of a first agent consisting of powder or liquid containing the compound having the pharmacological action, a second agent consisting of powder or liquid containing the crosslinking polysaccharide derivative, and a third agent consisting of liquid containing the pH adjuster. It is preferable that the second agent consists of powder in consideration of safety in the gel-forming property. It is preferable that the first agent containing the compound having the pharmacological action consists of liquid in consideration of easiness in mixing the first agent with the other agents when the sustained-release topically administered agent is used. It is preferable that the pH adjuster consists of liquid in consideration of easiness in mixing the pH adjuster with the other agents when the sustained-release topically administered agent is used. It is possible to prepare a mixed agent by composing both the compound powder having the pharmacological action and the crosslinking polysaccharide derivative of powder and mixing both with each other. It is also possible to compose the pH adjuster of liquid and add the compound having the pharmacological action thereto.

It is preferable that the first agent containing the compound having the pharmacological action consists of liquid, that the pH adjuster also consists of liquid, and that only the crosslinking polysaccharide derivative consists of powder. When the sustained-release topically administered agent is used, it is preferable that the sustained-release topically administered agent is of a type prepared by adding the crosslinking polysaccharide derivative to the liquid containing the compound having the pharmacological action and thereafter mixing the pH adjuster consisting of liquid with the mixture of the crosslinking polysaccharide derivative and the liquid containing the compound having the pharmacological action. It is preferable to form the sustained-release topically administered agent of the invention into a kit in consideration of convenience in using it.

It is preferable for the sustained-release topically administered agent of the invention to contain trehalose. It is preferable that the trehalose is present together with the crosslinking polysaccharide derivative. Therefore, mixed powder of the crosslinking polysaccharide derivative and the trehalose is preferable.

The trehalose is a kind of a non-reducing disaccharide having a form in which two molecules of D-glucose are (1,1)-linked. The trehalose includes three kinds of isomers different from each other in the glycosidic bond. The isomers include α, α-bond (mycose, α-D-glucopyranosyl α-D-glucopyranoside), α, β-bond (neo-trehalose, β-D-glucopyranosyl α-D-glucopyranoside), and β, β-bond (isotrehalose, β-D-glucopyranosyl β-D-glucopyranoside).

Although the isomers of the trehalose may be used singly or in a state where the above isomers are mixed, it is preferable to use the trehalose in which the glycosidic linkage consists of α, α-linkage. The trehalose in which the glycosidic linkage consists of the α, α-linkage is available most inexpensively. In the invention, anhydride and hydrous trehalose may be used.

The mixing amount of the crosslinking polysaccharide derivative contained in the sustained-release topically administered agent of the invention for the entire amount of the sustained-release topically administered agent is favorably 3% by weight (unless otherwise noted, % is hereinafter referred to as % by weight) to 35% and especially favorably 15% to 25% by weight.

The mixing amount of the compound having the pharmacological action contained in the sustained-release topically administered agent of the invention for the entire amount of the sustained-release topically administered agent is favorably 0.1% by weight (unless otherwise noted, % is hereinafter referred to as % by weight) to 90% and especially favorably 3% to 83% by weight, although the mixing amount thereof varies with the kind thereof.

The mixing amount of the pH adjuster contained in the sustained-release topically administered agent of the invention for the entire amount of the sustained-release topically administered agent is favorably 3% by weight (unless otherwise noted, % is hereinafter referred to as % by weight) to 50% and more favorably 15% to 40% by weight, although the mixing amount thereof varies with the kind thereof to be used.

The mixing amount of the trehalose contained in the sustained-release topically administered agent of the invention for the entire amount of the sustained-release topically administered agent is favorably 10% by weight (unless otherwise noted, % is hereinafter referred to as % by weight) to 50% and more favorably 15% to 40% by weight.

The sustained-release topically administered agent of the invention can further contain widely known additives within the range where the characteristics of the invention are not hindered. In this case, it is especially preferable to use biologically acceptable additives. Although the kind of the additive is not specifically limited, it is possible to exemplify a hardening catalyst, a filler, a plasticizer, a softening agent, a stabilizer, a dehydrating agent, a coloring agent, an anti-sagging agent, a thickener, a physical property adjuster, a reinforcing agent, a thixotropic agent, an deterioration preventive agent, a flame retarder, an antioxidant, an ultraviolet absorber, a pigment, a solvent, a carrier, an excipient, an antiseptic, a binder, a swelling agent, an isotonic agent, a solubilizing agent, a preservative, a buffering agent, and a diluent. The sustained-release topically administered agent is capable of containing one kind or two or more kinds of these additives.

Specific examples of the additive include water, physiological saline, pharmaceutically acceptable organic solvents, gelatin, collagen, polyvinyl alcohol, polyvinylpyrrolidone, carboxyvinyl polymers, sodium carboxymethyl cellulose, sodium polyacrylate, sodium alginate, water-soluble dextran, sodium carboxymethyl starch, pectin, methyl cellulose, ethyl cellulose, xanthan gum, gum arabic, tragacanth, casein, agar-agar, diglycerine, propylene glycol, polyethylene glycol, vaseline, paraffin, stearyl alcohol, stearic acid, human serum albumin (HAS), mannitol, sorbitol, lactose, PBS, nonionic surface-active agents, biodegradable polymers, serum-free media, surface-active agents acceptable as medical additives, and biologically acceptable physiological pH buffer solutions.

The polysaccharide derivative to be used for the sustained-release topically administered agent of the invention has the active ester group and the active hydrogen-containing group in one molecular chain of the polysaccharide. The active ester group and the active hydrogen-containing group react with each other to form the covalent bond and thereby the crosslinking structure. Because the main skeleton of the biodegradable gel to be formed is composed of not a material derived from a living body, but natural or artificial polysaccharide, the biodegradable gel allows a patient to avoid a risk of suffering from an infectious disease. The toxicity of component of the biodegradable gel and its decomposition products is weak. The component of the biodegradable gel and its decomposition products have a weak toxicity, and the main skeleton is composed of the polysaccharide. Thus, the biodegradable gel has a good biodegradability and bioabsorbability.

EXAMPLES

(Synthesis Example 1)

(1) Preparation of Raw Material Polysaccharide (acid-type polysaccharide)

Carboxymethyl dextran (acid-type CM dextran) was prepared as a raw material to be used as active esterified polysaccharide derivative.

After 10g of dextran (produced by Wako Pure Chemical Industries, Ltd., weight-average molecular weight: 25000) was dissolved in 62.5g of purified water, 62.5g of 36% sodium hydroxide solution (W/V) (sodium hydroxide produced by Wako Pure Chemical Industries, Ltd.) was added to the dextran -containing solution. Thereafter the solution was stirred at 25 degrees C for 90 minutes for dissolution.

Subsequently, 75 g of a 20% (w/v) monochloroacetic acid aqueous solution (monochloroacetic acid, manufactured by Wako Pure Chemical Industries, Ltd.) was added, and the mixture was stirred at 60.degree C. for 6 hours. Thereafter, the reaction solution was adjusted so as to have a pH of 1.0 by using 20% hydrochloric acid and then stirred at 25.degree C. for 2 hours. The reaction solution was added dropwise to 5 L of a 90% by volume ethanol aqueous solution (100% ethanol, manufactured by Wako Pure Chemical Industries, Ltd.), and a deposit was recovered by using a suction funnel. A deposit as obtained by using 3 L of a 90% by volume ethanol aqueous solution was washed and finally purged with ethanol, followed by drying under reduced pressure. There was thus prepared acid type CM dextran.

(2) Assay of Carboxy group or Carboxymethyl Group

With respect to the acid type CM dextran (raw material polysaccharide) as obtained above in (1), the determination of a carboxyl group or a carboxymethyl group contained therein was carried out. 0.2 g (A (g)) of the raw material polysaccharide was weighed and added to a mixed solution of 20 mL of a 0.1 mol/L sodium hydroxide aqueous solution and 10 mL of an 80% by volume methanol aqueous solution, and the mixture was stirred at 25.degree. C. for 3 hours. Three drops of a 1.0% (w/v) phenolphthalein/90% by volume ethanol aqueous solution as an indicator was added to the resulting solution, and acid-base back titration was carried out by using 0.05 mol/L sulfuric acid, thereby measuring a use amount (V1 mL) of the 0.05 mol/L sulfuric acid (phenolphthalein, manufactured by Wako Pure Chemical Industries, Ltd.). Furthermore, a use amount (V0 mL) of the 0.05 mol/L sulfuric acid was measured at a blank which was carried out in the same manner except for not adding the raw material saccharide.

An amount (B mmol/g) of a carboxyl group and a carboxymethyl group in the raw material saccharide was calculated according to the following numerical expression (1) and found to be 0.60 mmol/g. Incidentally, both the 0.1 mol/L sodium hydroxide aqueous solution and the 0.05 mol/L sulfuric acid as used had a strength of 1.00.

$$B = (V0-V1) \times 0.1 \div A \cdots\cdots (1)$$

A: Mass(g) of raw material polysaccharide

B: Amount (mmol/g) of carboxy group and carboxymethyl group

(3) Preparation of Active Esterified CM Dextran

For an active esterification reaction of the acid type CM dextran, DMSO as a reaction solvent, N-hydroxysuccinimide (NHS) (manufactured by Wako Pure Chemical Industries, Ltd.) as an electrophilic group introducing agent and 1-ethyl-3-dimethylaminopropylcarbodiimide hydrochloride (EDC) (manufactured by Wako Pure Chemical Industries, Ltd.) as a dehydration condensing agent were used, thereby preparing an active esterified polysaccharide (polysaccharide derivative).

2.0g of the acid-type CM dextran (amount of carboxymethyl group: 0.60 mmol/g) obtained in the above (1) was dissolved in 200g of the DMSO. Thereafter 2.8g (24.3 mmol) of the NHS and 4.6g (24.0 mmol) of the EDC were added to the mixture. The solution containing the above-described substances was stirred at 25 degrees C for 19 hours. After the reaction solution was dripped to 4L of a mixed solvent of acetone and methanol, a deposit was collected by using a suction funnel. After the obtained deposit was cleaned by using 4L of the mixed solvent of the acetone and the methanol, the deposit was dried under reduced pressure. Thereby active esterified CM Dextran was prepared.

(4) Calculation of introduction amount of NHS into Active Esterified Polysaccharide (Polysaccharide Derivative)

The introduction amount of the NHS into the active esterified CM dextran obtained in the above (3) was calculated in a manner described below. The calculated amount of the NHS was 0.70 mmol/g.

The introduction amount of the NHS into the active esterified CM dextran is the content thereof present per unit weight of the polysaccharide derivative.

In order to prepare a calibration curve of N-hydroxysuccinimide (NHS), NHS standard aqueous solutions of 0.1, 0.2, 0.5, 1.0 and 2.5 mM were prepared, respectively. 0.2 mL of a 2N sodium hydroxide aqueous solution was added to 1 mL of each of the NHS standard aqueous solutions, and the mixture was heated at 60 degree C. and stirred for 10 minutes. After allowing to it stand for cooling, 1.5 mL of 0.85 N sulfuric acid and 0.5 mL of a 0.5% $FeCl_3$/1 N hydrochloric acid solution were added, and an absorbance at an absorption wavelength of 500 nm was measured by using a spectrophotometer ($FeCl_3$, manufactured by Wako Pure Chemical Industries, Ltd.). A concentration and an absorbance of each of the NHS aqueous solutions were plotted on the X-axis and the Y-axis, respectively, and linear approximation was carried out, thereby obtaining the following numerical expression (2) for calculating an NHS concentration.

$$Y = \alpha X + \beta \cdots \cdots (2)$$

X: NHS concentration (mM)
Y: Absorbance at wavelength of 500nm
a=0.178 (slope)
$\beta$=0.021 (intercept)
r=0.995 (correlation coefficient)

The NHS concentration X (mM) is calculated on the basis of the absorbance.

Next, 0.01 g (C (g)) of the active esterified polysaccharide of (3) was weighed and added to 1 mL of pure water, and the mixture was stirred at 25 degree C. for 3 hours. Thereafter, 0.2 mL of a 2 N sodium hydroxide aqueous solution was added, and the mixture was heated at 60 degree C. and stirred for 10 minutes. After allowing it to stand for cooling to room temperature, 1.5 mL of 0.85 N hydrochloric acid was added. After removing insoluble matters from the resulting solution containing the insoluble matters by using filter cotton, 0.5 mL of a 0.5% $FeCl_3$/1 N hydrochloric acid solution was added, and an absorbance at an absorption wavelength of 500 nm was measured by using a spectrophotometer ($FeCl_3$, manufactured by Wako Pure Chemical Industries, Ltd.). When a measured value of absorbance exceeds the absorbance at the time when the concentration of the NHS standard solution is 5 mM, dilution with pure water was carried out (dilution ratio, H). The content of the NHS group (D mmol) in the active esterified polysaccharide was calculated from a measured value of absorbance by utilizing the foregoing numerical expression (2) for calculating the NHS concentration. Subsequently, an introduction amount of NHS in the active esterified polysaccharide was determined according to the following numerical expression (3).

$$\text{Introduction amount of NHS (mmol/g)} = (D \times H) \times 0.001 / C \quad \cdots (3)$$

(5) Self-Crosslinking Property of Active Esterified Polysaccharide Derivative

It was confirmed from the following test that the active esterified polysaccharide as obtained above is self-crosslinking. 0.2 g of the active esterified polysaccharide was weighed in a cleaned test tube having a capacity of 10 mL (LARBO LT-15100, manufactured by Terumo Kabushiki Kaisha), to which was then added 1 mL of pure water, followed by mixing. Next, 1 mL (pH: 8.3) of an 8.3% (w/v) sodium hydrogencarbonate aqueous solution (sodium hydrogencarbonate, manufactured by Wako Pure Chemical Industries, Ltd.) as a pH adjuster was added, followed by mixing at about 2,000 rpm for about one minute by using a test tube mixer (MT-31, manufactured by Yamato Scientific Co., Ltd.). The state of contents of the test tube was visually confirmed before and after the mixing. Thus, in the active esterified CM dextran, the contents in the test tube after mixing were a block-like material (hydrated gel) so that the active esterified CM dextran was judged to be "self-crosslinking".

(Example)

(Preparation of Sustained-Release Topically administered agent)

As the crosslinking polysaccharide derivative, a freeze-dried product of the NHS-modified carboxymethyl dextran of the synthesis example 1 was used. A freeze-dried product of trehalose was used. As the pH adjuster, dry sodium carbonate and dry sodium hydrogen carbonate were used. As a medication, ropivacaine hydrochloride hydrate (molecular weight: 328.88) was used.

After a mixture of the freeze-dried product of the NHS-modified carboxymethyl dextran and the trehalose were formed mixed with each other at a weight ratio of 1:1, the mixture was subjected to electron beam sterilization to obtain 2.5g of powder (powder A: first agent). By using the ropivacaine hydrochloride hydrate, 3.6ml of 59mg/ml a ropivacaine hydrochloric acid aqueous solution (liquid A: second agent) was prepared. A pH adjuster (liquid B: third agent) was prepared by adding 3.2g of the dry sodium carbonate and 1.2g of the sodium hydrogen carbonate to 23.5ml of water. Thereby the sustained-release topically administered agent (example) of the invention composed of the first agent, the second agent (liquid A), and the third agent (liquid B) was prepared.

(Experiment 1)

By using the sustained-release topically administered agent of the example, a test for confirming the sustained release property of the medication was conducted.

(Mixing Example 1)

By adding the first agent (2.5g of the mixture of the freeze-dried product of the NHS-modified carboxymethyl dextran and the trehalose) to the second agent (liquid A: 3.6m1 of 59mg/ml the ropivacaine hydrochloric acid aqueous solution) and dissolving the first agent in the second agent, a first liquid (mixture of the first agent and the second agent) was prepared. After 800$\mu$L of the first liquid was put in a 15mL falcon tube, 168$\mu$L of the third agent (liquid B, pH adjuster) was added to the first liquid. A biodegradable gel (gel 1) containing a medication was prepared by stirring the mixture of the first liquid and the third agent by a vortex mixer until a gel was formed. The period of time required for the gel to be formed was 15 seconds. The gel had a pH of 7.56 and cloudy white and was thus visible. The absorbance of the reflected light was measured by using a Spectrophotometer SA4000 produced by NIPPON DENSHOKU INDUSTRIES Co., ltd. L=76.60, a=-1.90, b=27.52.

(Mixing Example 2)

After 56$\mu$L of water was added to 112$\mu$E of the third agent (liquid B, pH adjuster) to form a prepared third agent (prepared liquid B, pH adjuster). 800$\mu$L of the first liquid same as that of the mixing example 1 was added to 168$\mu$L of the prepared third agent. Thereafter a biodegradable gel (gel 2) containing a medication was prepared by stirring the mixture of the prepared third agent and the first liquid by the vortex mixer until a gel was formed. The period of time required for the gel to be formed was 1 minute 8 seconds. The gel had a pH of 6.73 and cloudy and was thus visible.

(Mixing Example 3)

After 84$\mu$L of water was added to 84$\mu$E of the third agent (liquid B, pH adjuster) to form a prepared third agent (prepared liquid B, pH adjuster). 800$\mu$L of the first liquid same as that of the mixing example 1 was added to 168$\mu$L of the prepared third agent. Thereafter a biodegradable gel (gel 3) containing a medication was prepared by stirring the mixture of the prepared third agent and the first liquid by the vortex mixer until a gel was formed. The period of time required for the gel to be formed was two minutes. The gel had a pH of 6.42 and cloudy and was thus visible.

(Sustained-release property Confirmation Test 1)

After 2.5mL of 20300U/Lα-amylase (from human saliva) was measured exactly, a sodium lactate-containing transfusion solution was added thereto to prepare an amylase solution having a volume 50mL. The sodium lactate-containing transfusion solution contains 3.0g of sodium chloride, 0.150g of potassium chloride, 0.10g of calcium chloride hydrate, 3.10g of L-sodium lactate solution (L-sodium lactate: 1.55g) in 500ml of an active component.

After 205μL of the first liquid (mixture of first agent and second agent) was put in a screw tube bottle having a diameter of 2.1cm, 43μL of the third agent (liquid B, pH adjuster) was added to the first liquid so as to allow the mixture of the first liquid and the third agent to have the same mixing ratio as that of the mixing example 1. Thereafter the mixture was stirred by the vortex mixer until the gel was formed so as to prepare a biodegradable gel (gel 1) containing the medication.

Similarly, after 14μL of water was added to 29μL of the third agent (liquid B, pH adjuster) to form a prepared third agent (prepared liquid B, pH adjuster) so as to allow the prepared third agent to have the same mixing ratio as that of the mixing example 2. 205μL of the first liquid same as that of the mixing example 1 was added to 43μL of the prepared third agent so as to allow the mixture of the prepared third agent and the first liquid to have the same mixing ratio as that of the mixing example 2. Thereafter the mixture of the prepared third agent and the first liquid was stirred by the vortex mixer until a gel was formed so as to prepare a biodegradable gel (gel 2) containing the medication.

Similarly, after 22μL of water was added to 22μL of the third agent (liquid B, pH adjuster) to form the prepared third agent (prepared liquid B, pH adjuster) so as to allow the prepared third agent to have the same mixing ratio as that of the mixing example 3. 205μL of the first liquid same as that of the mixing example 1 was added to 44μL of the prepared third agent. Thereafter the mixture of the prepared third agent and the first liquid was stirred by the vortex mixer until a gel was formed so as to prepare a biodegradable gel (gel 3) containing the medication.

After 3mL of an amylase solution was added to the screw tube bottles containing gels 1 through 3 individually, the solution was immersed in a water bath whose temperature was set to 37 degrees C. 1ml of the amylase solution was collected after the lapse of 10 minutes, 30 minutes, one hour, two hours, three hours, six hours, 24 hours, 30 hours, 48 hours, and 72 hours. Accordance with the United Sates Pharmacopeia "Ropivacaine Hydrochloride Injection: Assay", peak areas of ropivacaine obtained from 20 μL of each of these samples were measured. From that, the overall release rate of ropivacaine was calculated. The results are shown in Table 1.

Table 1

| Elapsed time | Release rate (%) | | |
|---|---|---|---|
| | Gel 1 | Gel 2 | Gel 3 |
| 10 minutes | 2.94 | 6.52 | 10.58 |
| 30 minutes | 8.67 | 12.30 | 20.63 |
| 1 hour | 13.70 | 28.32 | 37.33 |
| 3 hours | 19.16 | 41.89 | 63.44 |
| 6 hours | 24.24 | 53.46 | 82.21 |
| 24 hours | 30.78 | 61.83 | 101.21 |
| 30 hours | 31.36 | 64.45 | 102.33 |
| 48 hours | 35.00 | 72.11 | 103.28 |
| 54 hours | 37.27 | 75.13 | 103.76 |
| 72 hours | 83.48 | 86.83 | 104.05 |

(Sustained-release property Confirmation Test 2)

After 9mL of PBS (phosphoric acid buffer solution, pH: 7.4) was added to 968μE of each of the screw tube bottles containing biodegradable gels (gels 1, 2, and 3) used in the sustained-release property Confirmation Test 1, each the screw tube bottles was immersed in a water bath whose temperature was set to 37 degrees C. After the lapse of 10 minutes, 30 minutes, one hour, two hours, three hours, six hours, 24 hours, and one week, 0.5mL of each solution was collected. The mobile phase was added to each sampled 0.5 mL to bring the total volume to 5 mL. Accordance with the United Sates Pharmacopeia "Ropivacaine Hydrochloride Injection: Assay", peak areas of ropivacaine obtained from 20 $\mu$L of each of these samples were measured. From that, the overall release rate of ropivacaine was calculated. The results are shown in Table 2.

Table 2

| Elapsed time | Release rate (%) | | |
|---|---|---|---|
| | Gel 1 | Gel 2 | Gel 3 |
| 10 minutes | 0.64 | 0.88 | 0.55 |
| 30 minutes | 0.88 | 2.04 | 1.50 |
| 1 hour | 1.00 | 2.87 | 2.16 |
| 2 hours | 1.08 | 3.17 | 2.77 |
| 3 hours | 1.09 | 3.38 | 3.00 |
| 6 hours | 1.69 | 3.45 | 3.54 |
| 24 hours | 2.18 | 4.62 | 4.05 |
| 1 week | 8.93 | 17.73 | 18.53 |

In a medical agent having an ordinary base, a medication is released in PBS. On the other hand, a medication was hardly released when the sustained-release topically administered agent of the invention was immersed in the PBS. It was confirmed that the medication was released from the sustained-release topically administered agent of the invention owing to the decomposition of the gel from the result of a release test (sustained-release property confirmation test 1) conducted by using an amylase solution as an immersion liquid and a release test (sustained-release property confirmation test 2) conducted by using the PBS as an immersion liquid. Because in the sustained-release topically administered agent of the invention, the release of the medication and the decomposition and disappearance of the gel occur at the same time, it does not occur that only the gel remains after the medication is all released.

(Experiment 2)

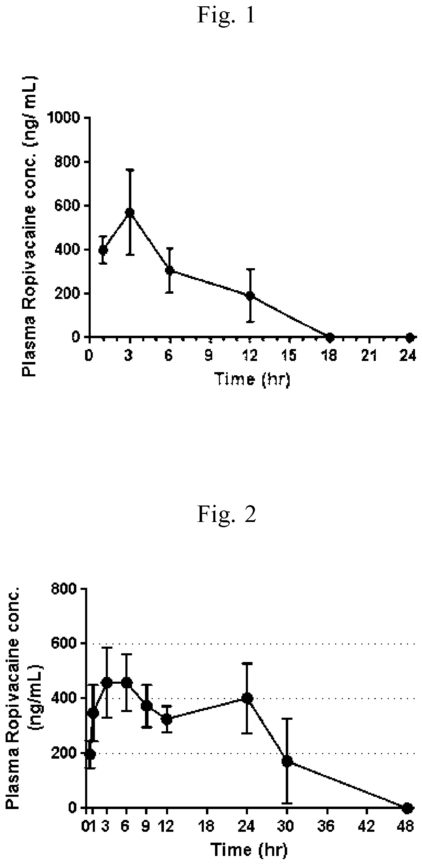

Using a rat, whether the medication-containing biodegradable gel remained was checked, and the concentration of the medication in plasma was measured.
After exposing the subcutaneous tissue (abdominal wall) to the outside by incising the abdomen of a male SD rat (body weight: 300g) under isoflurane anesthesia, the biodegradable gel (contained 8.75mg of ropivacaine hydrochloric acid, pH: 7.56) of the mixing example 1 was administered to the subcutaneous tissue (abdominal wall). Because the administered biodegradable gel was clouded, it was visually easy to recognize the part where the biodegradable gel was administered and the part where it was not administered.
Autopsy was carried out at time points of 1, 3, 6, 12, 18, 24, and 48 hours after the administration of the biodegradable gel (each time point n=3). Until before 12 hours lapsed after the administration of the biodegradable gel, remaining of the gels in all administration cases could be visually confirmed. When not less than 18 hours lapsed after the administration of the biodegradable gel, the gels did not remain.
The change in the concentration of the ropivacaine in the plasma was as shown in Fig. 1. The concentration thereof became highest when three hours lapsed after the administration of the biodegradable gel, became gradually lower thereafter, was about 200ng/mL when 12 hours lapsed after the administration of the biodegradable gel, and became less than the lowest limit in the quantitative determination when 18 hours lapsed after the administration of the biodegradable gel.

(Experiment 3)

Using rabbits, whether the medication-containing biodegradable gel remained was checked, and the concentration of the medication in plasma was measured.
After forming a subcutaneous pocket by incising and peeling the back of a female Kbl : JW rabbit (body weight: 3kg) off its body under isoflurane anesthesia, the biodegradable gel (contained 70mg of ropivacaine hydrochloric acid) of the mixing example 1 was administered to the pocket (six rabbits). Because the administered biodegradable gel was clouded, it was visually easy to recognize the part where the biodegradable gel was administered and the part where it was not administered.

Blood was collected at time points of 1, 3, 6, 9, 12, 24, 30, and 48 hours after the administration of the biodegradable gel. When 24, 48, and 72 hours lapsed after the administration of the biodegradable gel, autopsy was carried out for two rabbits. Until 24 hours lapsed after the administration of the biodegradable gel, remaining of the gels of all of the example solutions could be visually confirmed. When 48 hours and 72 hours lapsed after the administration of the biodegradable gel, the remaining of the gels could not be confirmed.

The change in the concentration of the ropivacaine in the plasma was as shown in Fig. 2. The concentration of the ropivacaine in the plasma became high rapidly after the administration of the biodegradable gel, became highest during the lapse of three to six hours after the administration of the biodegradable gel, remained at the same level thereafter, and became less than the lowest limit (10ng/ml) in the quantitative determination when 48 hours lapsed after the administration of the biodegradable gel.

The sustained-release topically administered agent of the invention forms the biodegradable gel containing the compound having the pharmacological action at a part where the sustained-release topically administered agent has been administered after mixing. Because the sustained-release topically administered agent gels, it remains at the part where it has been administered. At the same time, the compound having the pharmacological action contained in the biodegradable gel is gradually released because the collapse of the gel proceeds with the passage of time. Therefore, it is possible to administer the compound having the pharmacological action to the administered part for a considerable long term until the gel collapses. Because the release of the compound occurs with the decomposition of the gel, the compound and the gel disappear at substantially the same time and thus, the gel does not remain. The gel formed has good adhesion to a biological surface and excellent flexibility.

**Claims**

1. A sustained-release agent for topical administration, in the form of a kit, to be mixed and comprising:

   a compound having a pharmacological action;
   a crosslinking polysaccharide derivative having at least one active ester group, introduced into a side chain of a polysaccharide, which is capable of reacting with an active hydrogen-containing group, and being capable of forming a crosslinked substance by a covalent bond between said active ester group and said active hydrogen-containing group;
   a pH adjuster, not mixed with said crosslinking polysaccharide derivative, for adjusting a pH condition suitable for forming a biodegradable gel containing said compound when said sustained-release agent is mixed.

2. The sustained-release agent for topical administration according to claim 1, comprising:

   a first agent consisting of liquid containing the compound having a pharmacological action,
   a second agent consisting of powder containing the crosslinking polysaccharide derivative and
   a third agent consisting of liquid containing the pH adjuster.

3. A biodegradable gel formed by mixing the ingredients of the sustained-release agent according to claim 1 or 2, having a pH of not less than five when said sustained-release agent is mixed.

4. The biodegradable gel according to claim 3, wherein said pH is 6.4 to 12.

5. The biodegradable gel according to claim 3 or 4, wherein a release rate of said compound during 1 hour from a formation of said biodegradable gel is 10% or more and a release rate of said compound during 6 hours from said formation of the biodegradable gel is less than 70%.

6. The biodegradable gel according to any one of claims 3 through 5, wherein said compound having said pharmacological action is at least one selected from among a group consisting of a local anesthetic medication, antibiotics, antimicrobial agents, antifungal agents, antiviral agents, anti-inflammatory agents, vasoconstricting agents, steroid hormone agent, antihistamines, prostaglandin, and anti-cancer drugs.

7. The biodegradable gel according to any one of claims 3 to 6, wherein said compound having said pharmacological action is a local anesthetic medication and said local anesthetic medication is at least one selected from among a group consisting of ropivacaine, bupivacaine, levobupivacaine, lidocaine, procaine, tetracaine, cocaine, benzocaine, mepivacaine, prilocaine, dibucaine, chloroprocaine, and etidocaine or salts of these local anesthetic medications.

8. The biodegradable gel according to any one of claims 3 through 7, wherein said pH adjuster consists of an inorganic base or an organic base and consists of any one of sodium hydroxide, sodium carbonate, sodium hydrogen carbonate, a phosphoric acid-based buffer agent, and an acetic acid-ammonia based buffer agent.

9. The biodegradable gel according to any one of claims 3 through 8, which has visibility owing to a phenomenon that said biodegradable gel becomes suspended.

10. The biodegradable gel according to claim 9, which becomes cloudy when the ingredients of said sustained-release topically administered agent are mixed.


**Patentansprüche**

1. Mittel mit verzögerter Freisetzung zur topischen Verabreichung in Form eines zu mischenden Kits, umfassend:

    eine Verbindung mit pharmakologischer Wirkung;
    ein vernetzendes Polysaccharidderivat mit mindestens einer in eine Seitenkette eines Polysaccharids eingebauten aktiven Estergruppe, die mit einer aktiven wasserstoffhaltigen Gruppe reagieren kann und die in der Lage ist, durch eine kovalente Bindung zwischen der aktiven Estergruppe und der aktiven wasserstoffhaltigen Gruppe eine vernetzte Substanz zu bilden;
    ein pH-Einstellmittel, das nicht mit dem vernetzenden Polysaccharidderivat vermischt ist, zum Einstellen eines pH-Zustandes, der zur Bildung eines die Verbindung enthaltenden biologisch abbaubaren Gels geeignet ist, wenn das Mittel mit verzögerter Freisetzung gemischt wird.

2. Mittel mit verzögerter Freisetzung zur topischen Verabreichung nach Anspruch 1, umfassend:

    ein erstes Mittel, das aus einer die Verbindung mit pharmakologischer Wirkung enthaltenden Flüssigkeit besteht,
    ein zweites Mittel, das aus einem das vernetzende Polysaccharidderivat enthaltenden Pulver besteht, und
    einem dritten Mittel, das aus einer das pH-Einstellmittel enthaltenden Flüssigkeit besteht.

3. Biologisch abbaubares Gel, gebildet durch Mischen der Bestandteile des Mittels mit verzögerter Freisetzung nach Anspruch 1 oder 2, mit einem pH-Wert nicht kleiner als 5, wenn das Mittel mit verzögerter Freisetzung gemischt wird.

4. Biologisch abbaubares Gel nach Anspruch 3, wobei der pH-Wert 6,4 bis 12 beträgt.

5. Biologisch abbaubares Gel nach Anspruch 3 oder 4, wobei eine Freisetzungsrate der Verbindung innerhalb von 1 Stunde seit Bildung des biologisch abbaubaren Gels 10% oder mehr beträgt und eine Freisetzungsrate der Verbindung innerhalb von 6 Stunden seit Bildung des biologisch abbaubaren Gels weniger als 70% beträgt.

6. Biologisch abbaubares Gel nach einem der Ansprüche 3 bis 5, wobei die Verbindung mit pharmakologischer Wirkung mindestens eine Verbindung ist, die aus einer aus einem Lokalanästhetikum, Antibiotika, antimikrobiellen Mitteln, Antimykotika, Virostatika, entzündungshemmenden Mitteln, gefäßverengenden Mitteln, Mitteln mit Steroidhormonen, Antihistaminika, Prostaglandin und Antikrebsmitteln bestehenden Gruppe ausgewählt ist.

7. Biologisch abbaubares Gel nach einem der Ansprüche 3 bis 6, wobei die Verbindung mit pharmakologischer Wirkung ein Lokalanästhetikum ist und das Lokalanästhetikum mindestens ein Medikament ist, das aus einer aus Ropivacain, Bupivacain, Levobupivacain, Lidocain, Procain, Tetracain, Kokain, Benzocain, Mepivacain, Prilocain, Dibucain, Chlorprocain und Etidocain oder Salzen dieser Lokalanästhetika bestehenden Gruppe ausgewählt ist.

8. Biologisch abbaubares Gel nach einem der Ansprüche 3 bis 7, wobei das pH-Einstellmittel aus einer anorganischen Base oder einer organischen Base besteht und aus einem von Natriumhydroxid, Natriumcarbonat, Natriumhydrogencarbonat, einem Puffermittel auf Basis von Phosphorsäure und einem Puffermittel auf Basis von Essigsäure und Ammoniak besteht.

9. Biologisch abbaubares Gel nach einem der Ansprüche 3 bis 8, das aufgrund eines Phänomens durchsichtig ist, wonach das biologisch abbaubare Gel suspendiert wird.

10. Biologisch abbaubares Gel nach Anspruch 9, das trüb wird, wenn die Bestandteile des topisch verabreichten Mittels mit verzögerter Freisetzung vermischt werden.

**Revendications**

1. Agent à libération prolongée pour une administration topique, sous la forme d'un kit, destiné à être mélangé et comprenant :

   un composé présentant une action pharmacologique ;
   un dérivé polysaccharide de réticulation présentant au moins un groupe ester actif, introduit dans une chaîne latérale d'un polysaccharide, qui est capable de réagir avec un groupe contenant un hydrogène actif, et capable de former une substance réticulée par une liaison covalente entre ledit grouper ester actif et ledit groupe contenant un hydrogène actif ;
   un régulateur de pH, non mélangé audit dérivé polysaccharide de réticulation, pour régler une condition de pH adaptée pour former un gel biodégradable contenant ledit composé lorsque ledit agent à libération prolongée est mélangé.

2. Agent à libération prolongée pour une administration topique selon la revendication 1, comprenant :

   un premier agent constitué d'un liquide contenant le composé présentant une action pharmacologique,
   un deuxième agent constitué d'une poudre contenant le dérivé polysaccharide de réticulation et
   un troisième agent constitué d'un liquide contenant le régulateur de pH.

3. Gel biodégradable formé par mélange des ingrédients de l'agent à libération prolongée selon la revendication 1 ou 2, présentant un pH non inférieur à cinq lorsque ledit agent à libération prolongée est mélangé.

4. Gel biodégradable selon la revendication 3, dans lequel ledit pH est 6,4 à 12.

5. Gel biodégradable selon la revendication 3 ou 4, dans lequel un taux de libération dudit composé pendant 1 heure à partir d'une formation dudit gel biodégradable est 10 % ou plus et un taux de libération dudit composé pendant 6 heures à partir de ladite formation du gel biodégradable est inférieur à 70 %.

6. Gel biodégradable selon l'une quelconque des revendications 3 à 5, dans lequel ledit composé présentant ladite action pharmacologique est au moins un sélectionné dans un groupe constitué d'un médicament anesthésique local, d'antibiotiques, d'agents antimicrobiens, d'agents antifongiques, d'agents antiviraux, d'agents antiinflammatoires, d'agents vasoconstricteurs, d'agents de stéroïde hormonal, d'antihistaminiques, de prostaglandine, et de médicaments anticancéreux.

7. Gel biodégradable selon l'une quelconque des revendications 3 à 6, dans lequel ledit composé présentant ladite action pharmacologique est un médicament anesthésique local et ledit médicament anesthésique local est au moins un sélectionné dans un groupe constitué de la ropivacaïne, bupivacaïne, levobupicaïne, lidocaïne, procaïne, tétracaïne, cocaïne, benzocaïne, mépivacaïne, prilocaïne, dibucaïne, chloroprocaïne, et étidocaïne ou des sels de ces médicaments anesthésiques locaux.

8. Gel biodégradable selon l'une quelconque des revendications 3 à 7, dans lequel ledit régulateur de pH est constitué d'une base inorganique ou d'une base organique et est constitué de n'importe quel parmi l'hydroxyde de sodium, le carbonate de sodium, l'hydrogénocarbonate de sodium, un tampon à base d'acide phosphorique, et un tampon à base d'ammoniac d'acide acétique.

9. Gel biodégradable selon l'une quelconque des revendications 3 à 8, qui présente une visibilité en raison d'un phénomène selon lequel ledit gel biodégradable devient en suspension.

10. Gel biodégradable selon la revendication 9, qui devient trouble lorsque les ingrédients dudit agent administré de manière topique à libération prolongée sont mélangés.

Fig. 1

Fig. 2

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2011508788 W
- WO 2009129149 A
- JP 2013523693 W
- WO 2011121074 A
- JP 2013523694 W
- WO 2011121082 A
- US 2013079371
- JP 2006523731 W
- WO 200492223 A
- JP 2007521225 W
- WO 2005009408 A
- WO 2005087289 A
- US 7737214 B
- WO 2010043979 A2
- WO 2012102210 A
- WO 2010043979 A

**Non-patent literature cited in the description**

- *Biochemistry,* 1975, vol. 14 (7), 1535-1541
- Poly(ethylene Glycol) Chemistry: Biotechnical and Biomedical Applications. Plenum Press, 1992